# EUROPEAN PATENT APPLICATION

(11) **EP 4 302 597 A1**
(43) Date of publication of application: **10.01.2024**
(21) Application number: 21929053.3
(22) Date of filing: 04.03.2021
(51) Int. Cl.: A01K 67/033

(54) **REARING DEVICE**

(71) Applicant: JTEKT Corporation, Kariya-shi, Aichi-ken, 448-8652 (JP); Tokushima University, Tokushima-shi, Tokushima 770-8501 (JP)
(72) Inventor: MIURA, Nozomu, Kariya-shi, Aichi 448-8652 (JP); MURATA, Yasuhiro, Kariya-shi, Aichi 448-8652 (JP); KAWAI, Shigekazu, Kariya-shi, Aichi 448-8652 (JP); WATANABE, Takahito, Tokushima-shi, Tokushima 770-8501 (JP); MITO, Taro, Tokushima-shi, Tokushima 770-8501 (JP)
(74) Representative: TBK
(86) International application number: PCT/JP2021/008447
(87) International publication number: WO 2022/185480

(57) **Abstract**

Provided is a breeding apparatus that can facilitate disposition and collection of a spawning bed. A breeding apparatus (1) includes a breeding area forming member (10) that forms breeding areas (A1, A2) for organisms and is open at least at the bottoms of the breeding areas (A 1, A2), and a spawning bed sheet (41) that forms the bottom surfaces of the breeding areas (A1, A2), has a belt shape, and is provided so as to be movable in a longitudinal direction of the belt shape relative to the breeding area forming member (10).

## Description

### TECHNICAL FIELD

The present invention relates to a breeding apparatus.

### BACKGROUND ART

Patent Document 1 describes a cricket breeding apparatus. In this breeding apparatus, spawning, hatching, and breeding are all performed in a breeding case. A spawning medium composed of crushed soil and sand is placed in a spawning container disposed in the breeding case, and crickets spawn on the spawning medium.

Patent Document 2 describes a facility for breeding insects. The facility has a spawning container, a baby larva container, a pupation container, and the like, and includes a belt conveyor for conveying each container. A spawning structure including a structure that allows entry of an ovipositor of a cricket is fixed to the spawning container. Eggs are collected by conveying the spawning container on the conveyor.

### Related Art Documents

### Patent Documents

Patent Document 1: Japanese Unexamined Patent Application Publication No. 10-191834 (JP 10-191834 A)
Patent Document 2: Japanese Unexamined Patent Application Publication (Translation of PCT Application) No. 2018-509167 (JP 2018-509167 A)

### SUMMARY OF THE INVENTION

### Problem to be Solved by the Invention

In the breeding case described in Patent Document 1, when collecting spawned eggs, a person needs to take out the spawning container from the breeding case and take out the spawning medium from the spawning container. Therefore, it takes time and effort to collect eggs. When disposing the spawning container, a person needs to attach the spawning medium to the spawning container and dispose the spawning container in the breeding case.

In the facility described in Patent Document 2, eggs are collected by being conveyed together with the spawning container to which the spawning structure is fixed. It is then necessary to remove the spawning structure from the spawning container. When disposing the spawning container in a spawning area, it is necessary to attach the spawning structure to the spawning container in advance.

An object of the present invention is to provide a breeding apparatus that can facilitate disposition and collection of a spawning bed.

### Means for Solving the Problem

A breeding apparatus includes a breeding area forming member that forms a breeding area for an organism and is open at least at a bottom of the breeding area, and a spawning bed sheet that forms a bottom surface of the breeding area, has a belt shape, and is provided so as to be movable in a longitudinal direction of the belt shape relative to the breeding area forming member.

The spawning bed sheet is carried into the bottom position of the breeding area from the outside of the breeding area, and is carried to the outside from the bottom position of the breeding area. That is, the spawning bed can be disposed and collected by moving the spawning bed sheet. By repeating the movement of the spawning bed sheet, the spawning bed can be disposed and collected repeatedly. Thus, the spawning bed can be disposed and collected very easily.

### BRIEF DESCRIPTION OF THE DRAWINGS

[FIG. 1] FIG. 1 is a front view of a breeding apparatus.
[FIG. 2] FIG. 2 is a diagram viewed in a direction II in FIG. 1, that is, a top view of the breeding apparatus.
[FIG. 3] FIG. 3 is a sectional view taken along a line III-III in FIG. 2.
[FIG. 4] FIG. 4 is a sectional view taken along a line IV-IV in FIG. 2.
[FIG. 5] FIG. 5 is a sectional view taken along a line V-V in FIG. 3.
[FIG. 6] FIG. 6 is an enlarged view of a portion VI in FIG. 3.
[FIG. 7] FIG. 7 is a diagram of a breeding apparatus in another example and corresponds to FIG. 5.

### MODES FOR CARRYING OUT THE INVENTION

### (1. Breeding Target Organisms)

Organisms to be bred in a breeding apparatus are small organisms such as arthropods. The breeding target organisms are, for example, organisms to be used for food, feed, or research. Insects among the breeding target organisms include crickets, locusts, grasshoppers, and the like. Among the insects, crickets and locusts belonging to orthoptera will be described as preferable examples of the breeding target organisms in this example.

### (2. Overview of Breeding Apparatus)

The breeding apparatus in this example breeds the breeding target organisms during a spawning period. That is, the breeding apparatus breeds grown breeding target organisms while they spawn eggs. In particular, the breeding apparatus is intended for collection of spawned eggs. The breeding apparatus in this example can be coupled to an apparatus for hatching out eggs and growing hatched larvae, thereby serving as an apparatus for breeding the breeding target organisms over the entire lives.

### (3. Configuration of Breeding Apparatus 1)

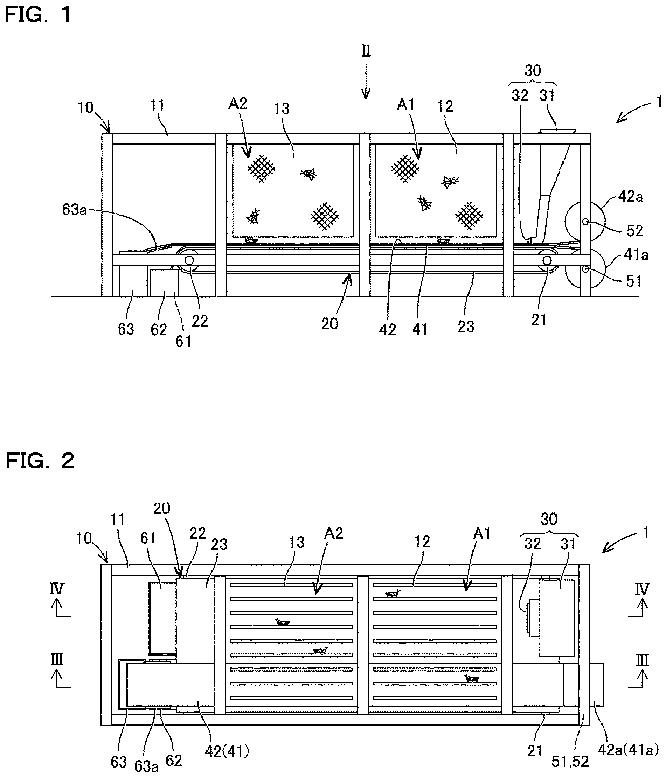

The configuration of a breeding apparatus 1 will be described with reference to FIGS. 1 to 5. As shown in FIG. 1, the breeding apparatus 1 includes a breeding area forming member 10, a conveyor 20, a feeding device 30, a spawning bed sheet 41, a protection sheet 42, a spawning bed sheet holding member 51, a protection sheet holding member 52, a leftover food collection container 61, a spawning bed sheet collection container 62, and a protection sheet collection container 63. Although illustration is omitted, the breeding apparatus 1 further includes a control device that controls the conveyor 20 and the feeding device 30.

The breeding area forming member 10 forms breeding areas A1, A2 for breeding the breeding target organisms. The breeding area forming member 10 includes a frame 11 and breeding area facilities 12, 13.

The frame 11 is fixed to an installation surface, and is formed in, for example, a rectangular parallelepiped shape with its longitudinal direction corresponding to a horizontal direction. The shape of the frame 11 can be set arbitrarily. The frame 11 forms the breeding areas A1, A2 adjacent in the horizontal direction. In this example, two breeding areas A1, A2 are formed, but only one breeding area may be formed, or three or more breeding areas may be formed. The breeding areas A1, A2 are positioned away from the installation surface in an upward direction and are open at least at the bottoms. In this example, the breeding areas A1, A2 are also open at the sides. The breeding areas A1, A2 may be closed at the sides and tops.

The breeding area facilities 12, 13 are disposed in the breeding areas A 1,A2 and include a plurality of stay members. The stay members are fixed to the frame 11 to extend in a vertical direction, and the breeding target organisms can stay on the stay members. The stay members function as hiding places for the organisms.

The stay members are, for example, plate members and are arranged to face each other in the horizontal direction. In this example, the stay member is formed in a flat plate shape, but may be formed in a wavy shape. In this example, the stay member extends in the longitudinal direction of the frame 11 (lateral direction in FIG. 2). That is, the plurality of stay members is arranged to face each other in a transverse direction of the frame 11 (vertical direction in FIG. 2). The stay members may be arranged to face each other in the longitudinal direction of the frame 11 or may be arranged to face each other in an oblique direction.

The stay member is made of metal such as iron or aluminum, resin, wood, paper, cloth, or the like. In the stay member, a large number of pores or projections and recesses are formed over the entire surface so that the stay member serves as a foothold for organisms such as insects. For example, the stay member is made of a punching metal or a wire mesh. For example, the stay member has a thickness of 1 to 5 [mm], and has pores with a diameter of about 1 [mm] and a pitch of about 2 [mm]. To function as hiding places, the interval between adjacent stay members is, for example, 10 to 30 [mm].

The conveyor 20 is disposed below the breeding areas A1, A2 such that its conveyance direction corresponds to the longitudinal direction of the frame 11. The conveyor 20 constitutes the bottom surfaces of the breeding areas A1, A2. The conveyor 20 is, for example, a belt conveyor. A roller conveyor or the like can be employed as the conveyor 20 instead of the belt conveyor.

The conveyor 20 includes a pair of support rollers 21, 22 and an endless belt 23 looped around the pair of support rollers 21, 22. The support rollers 21, 22 are fixed to the frame 11. By driving at least one of the pair of support rollers 21, 22 to rotate, the belt 23 can be moved continuously. The conveyance direction of the conveyor 20 is the movement direction of the upper surface of the belt 23. In FIG. 1, the conveyance direction of the conveyor 20 is a direction from right to left. The width of the belt 23 is approximately the same as the width of each of the breeding areas A1, A2, and the belt 23 constitutes the bottom surfaces of the breeding areas A1, A2.

The upper surface of the belt 23 is positioned slightly away in a downward direction from the lower ends of the stay members that constitute the breeding area facilities 12, 13. Therefore, the belt 23 does not come into contact with the stay members when the belt 23 moves. The distance between the upper surface of the belt 23 and the lower end of the stay member is such that the breeding target organism can transfer between them.

The feeding device 30 is a device for feeding the organisms bred in the breeding areas A1, A2. The feeding device 30 is disposed on an upstream side of the frame 11 in the conveyance direction of the conveyor 20. The feeding device 30 supplies food to the upper surface of the belt 23 of the conveyor 20.

The feeding device 30 includes a hopper 31 that holds food, and a rotary brush 32 that switches a state in which food is supplied from an outlet of the hopper 31 and a state in which the supply is stopped. The hopper 31 is formed such that the passage area decreases downward from the upper end opening. The rotary brush 32 is provided at the lower end opening of the hopper 31. The rotary brush 32 supplies food to the upper surface of the belt 23 of the conveyor 20 when the rotary brush 32 is driven to rotate, and stops supplying food when the rotation is stopped.

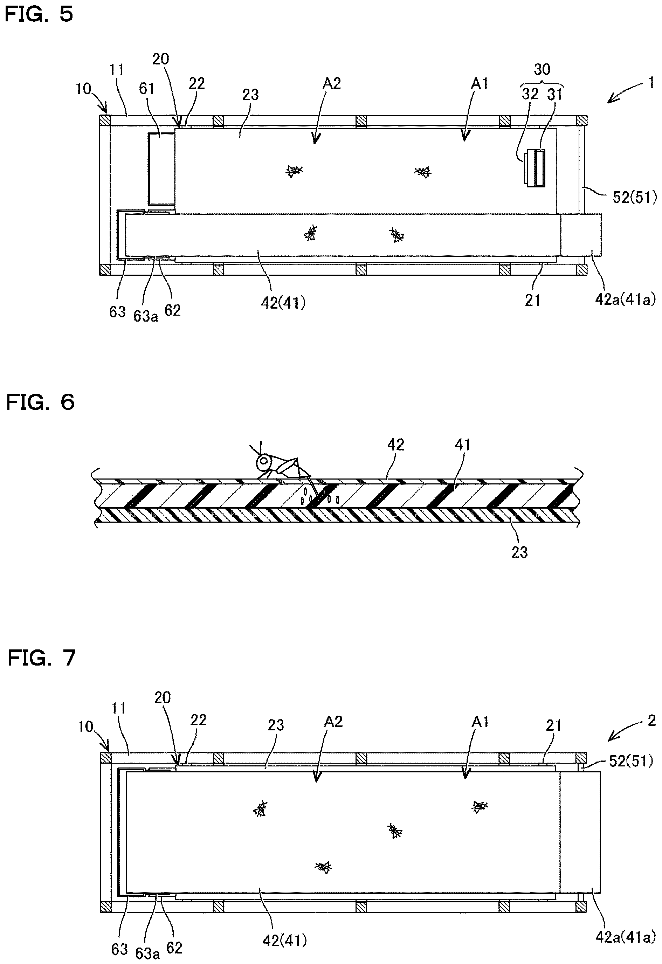

As shown in FIGS. 2 and 5, the food supply port of the feeding device 30 is positioned closer to one side of the conveyor 20 in its width direction. In this example, the food supply port of the feeding device 30 is positioned on a right side in a traveling direction assuming that the conveyance direction of the conveyor 20 is a forward direction. When the belt 23 is conveyed after the food is supplied from the feeding device 30 to the upper surface of the belt 23, the food on the upper surface of the belt 23 is supplied to the breeding areas A1, A2. Thus, the conveyor 20 has a part in the width direction (upper part in FIGS. 2 and 5) as a food supply area.

The spawning bed sheet 41 is a sheet capable of functioning as a spawning bed where the breeding target organisms spawn. It is necessary that the spawning bed can hold eggs as well as keeping moisture. Therefore, the spawning bed sheet 41 is a sheet that absorbs moisture, and is made of, for example, paper or cloth. The spawning bed sheet 41 has a thickness enough to hold spawned eggs. The spawning bed sheet 41 can be pierced with an ovipositor of the breeding target organism, and holds eggs sent out from the ovipositor of the organism.

In this example, the spawning bed sheet 41 is formed in a continuous belt shape. The spawning bed sheet 41 forms at least the bottom surfaces of the breeding areas A1, A2. Specifically, the spawning bed sheet 41 is laid over and in contact with the upper surface of the belt 23 of the conveyor 20, and is disposed such that the longitudinal direction of the belt of the spawning bed sheet 41 coincides with the conveyance direction of the conveyor 20.

Since the spawning bed sheet 41 is in contact with the upper surface of the belt 23 of the conveyor 20, the spawning bed sheet 41 can move in the longitudinal direction of the belt of the spawning bed sheet 41 relative to the breeding area forming member 10 as the conveyor 20 is conveyed. That is, the spawning bed sheet 41 moves from upstream to downstream of the conveyor 20 relative to the breeding area forming member 10 by a conveyance force of the conveyor 20. Thus, the conveyor 20 exhibits a function of conveying the spawning bed sheet 41 with the spawning bed sheet 41 placed on the conveyor 20.

The spawning bed sheet 41 is conveyed by a frictional force between the belt 23 of the conveyor 20 and the spawning bed sheet 41. Therefore, the surface of the belt 23 of the conveyor 20 and the surface of the spawning bed sheet 41 are made of materials having frictional forces enough to transmit the conveyance force.

As shown in FIGS. 2 and 5, the spawning bed sheet 41 is positioned closer to the other side of the conveyor 20 in the width direction. In this example, the spawning bed sheet 41 is positioned on a side different from the food supply area where food is supplied by the feeding device 30, that is, on a left side in the traveling direction (lower side in FIGS. 2 and 5) assuming that the conveyance direction of the conveyor 20 is the forward direction. That is, the spawning bed sheet 41 is positioned on the upper surface of the belt 23 at a position different from the food supply area. In other words, the upper surface of the belt 23 is divided into the food supply area and a spawning bed area in the width direction of the conveyor 20.

In general, when food is left at a moist position for a long period, the quality of the food deteriorates due to mold or the like. The spawning bed needs to keep moisture as described above. By defining the food supply area and the spawning bed area separately, it is possible to suppress the deterioration in the quality of the food and secure a satisfactory spawning bed.

The protection sheet 42 is a sheet that protects the spawning bed sheet 41. If the spawning bed sheet 41 is exposed on the surface, unwanted matter including at least a part of dust, food, excrement, dead organisms, and the like may adhere to the surface of the spawning bed sheet 41. The unwanted matter needs to be removed from the spawning bed sheet 41 in the subsequent process. The protection sheet 42 capable of protecting the spawning bed sheet 41 is used to prevent the unwanted matter from adhering to the spawning bed sheet 41.

The protection sheet 42 is laid over the upper surface of the spawning bed sheet 41. The protection sheet 42 allows passage of the ovipositor of the organism while restricting passage of the unwanted matter. The material and thickness of the protection sheet 42 are set so that the organism spawns on the spawning bed sheet 41 without spawning on the protection sheet 42. For example, the protection sheet 42 is formed to be thinner than the spawning bed sheet 41. For example, the protection sheet 42 is sufficiently thinner than the length of the ovipositor of the organism. The protection sheet 42 is made of a resin film, paper, cloth, or the like. The protection sheet 42 may have holes sufficiently smaller than the size of the unwanted matter, or may have no holes.

In this example, the protection sheet 42 is formed in a continuous belt shape similarly to the spawning bed sheet 41. The width of the protection sheet 42 is equal to or larger than that of the spawning bed sheet 41. The protection sheet 42 forms at least the bottom surfaces of the breeding areas A1, A2. The upper surface of the protection sheet 42 is exposed and the protection sheet 42 forms the bottom surfaces of the breeding areas A1, A2.

The protection sheet 42 is laid over the spawning bed sheet 41. That is, the protection sheet 42 is placed in contact with the upper surface of the spawning bed sheet 41. Similarly to the spawning bed sheet 41, the protection sheet 42 is disposed such that the longitudinal direction of the belt of the protection sheet 42 coincides with the conveyance direction of the conveyor 20.

Since the protection sheet 42 is in contact with the upper surface of the spawning bed sheet 41, the protection sheet 42 can move in the longitudinal direction of the belt of the protection sheet 42 relative to the breeding area forming member 10 together with the spawning bed sheet 41 as the conveyor 20 is conveyed. That is, similarly to the spawning bed sheet 41, the protection sheet 42 moves from upstream to downstream of the conveyor 20 relative to the breeding area forming member 10 by the conveyance force of the conveyor 20. Thus, the conveyor 20 exhibits a function of conveying the protection sheet 42 in addition to the spawning bed sheet 41.

The protection sheet 42 is not bonded to the spawning bed sheet 41. The protection sheet 42 is conveyed by a frictional force between the spawning bed sheet 41 and the protection sheet 42. Therefore, the surface of the spawning bed sheet 41 and the surface of the protection sheet 42 are made of materials having frictional forces enough to transmit the conveyance force.

It is necessary that the organisms be aware of the presence of the spawning bed sheet 41 under the protection sheet 42. Therefore, it is appropriate to form a plurality of holes in the protection sheet 42 and expose a part of the upper surface of the spawning bed sheet 41 through the holes in the protection sheet 42. Thus, the organisms can grasp the humidity and spawn on the spawning bed sheet 41. The protection sheet 42 itself may contain moisture. Thus, an environment in which the organisms easily spawn is formed, and eggs are spawned on the spawning bed sheet 41 by piercing with the ovipositor.

The spawning bed sheet holding member 51 is disposed on the upstream side in the conveyance direction of the conveyor 20, and holds a spawning bed sheet 41 a before the supply to the breeding areas A1, A2. The held spawning bed sheet 41a is pulled out from the spawning bed sheet holding member 51 by the conveyance force of the conveyor 20 and therefore supplied to positions below the breeding areas A1, A2.

For example, the spawning bed sheet holding member 51 includes a spindle fixed to the frame 11 on the upstream side in the conveyance direction of the conveyor 20, and a bobbin rotatably supported by the spindle. The spawning bed sheet 41a is wound around the outer peripheral surface of the bobbin. That is, the spawning bed sheet 41a wound around the bobbin constituting the spawning bed sheet holding member 51 is pulled out from the bobbin. The pulled out spawning bed sheet 41a is placed on the upper surface of the belt 23 of the conveyor 20 to form the spawning bed sheet 41 serving as the bottom surfaces of the breeding areas A1, A2. Since the bobbin is rotatable, it is possible to easily pull out the spawning bed sheet 41a from the bobbin of the spawning bed sheet holding member 51 by the conveyance force of the conveyor 20.

The protection sheet holding member 52 is disposed on the upstream side in the conveyance direction of the conveyor 20, and holds a protection sheet 42a before the supply to the breeding areas A1, A2. The held protection sheet 42a is pulled out from the protection sheet holding member 52 by the conveyance force of the conveyor 20 and therefore supplied to positions below the breeding areas A1, A2.

For example, the protection sheet holding member 52 includes a spindle fixed to the frame 11 on the upstream side in the conveyance direction of the conveyor 20, and a bobbin rotatably supported by the spindle. In particular, the spindle of the protection sheet holding member 52 is fixed above the spindle of the spawning bed sheet holding member 51 in the frame 11. The protection sheet 42a is wound around the outer peripheral surface of the bobbin.

That is, the protection sheet 42a wound around the bobbin constituting the protection sheet holding member 52 is pulled out from the bobbin. The pulled out protection sheet 42a is laid over the upper surface of the spawning bed sheet 41 on the upper surface of the belt 23 of the conveyor 20 to protect the spawning bed sheet 41 serving as the bottom surfaces of the breeding areas A1, A2. Since the bobbin is rotatable, it is possible to easily pull out the protection sheet 42a from the bobbin of the protection sheet holding member 52 by the conveyance force of the conveyor 20.

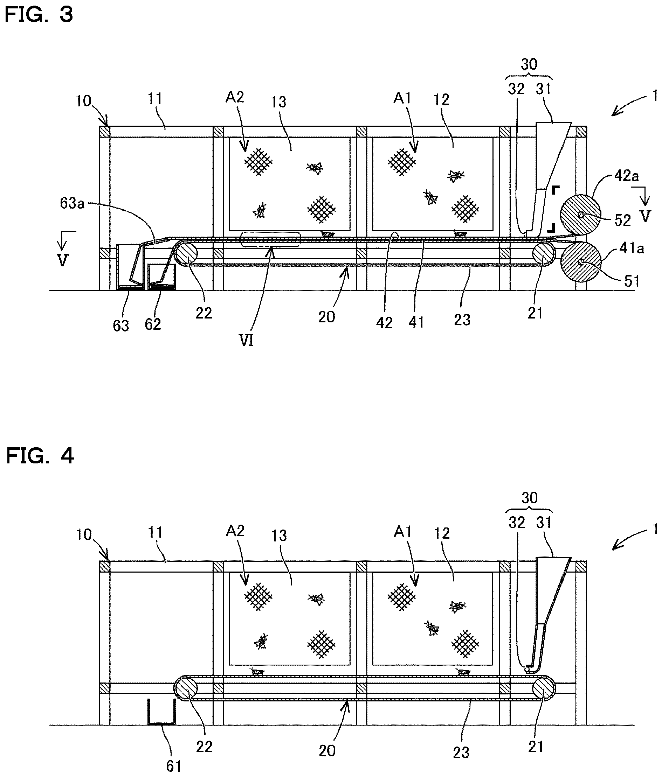

As shown in FIGS. 2 and 4, the leftover food collection container 61 is disposed on a downstream side in the conveyance direction of the conveyor 20, and collects leftover food supplied from the feeding device 30. The leftover food collection container 61 is positioned on the downstream of the conveyor 20 closer to one side of the conveyor 20 in the width direction (upper side in FIGS. 2 and 5). Thus, the upper surface of the belt 23 of the conveyor 20 can always be kept clean. The leftover food collection container 61 also collects, together with the leftover food, unwanted matter such as dust, excrement, and dead organisms in the food supply area on the upper surface of the belt 23.

As shown in FIGS. 1 to 3 and 5, the spawning bed sheet collection container 62 is disposed on the downstream side in the conveyance direction of the conveyor 20, and collects the spawning bed sheet 41 discharged from the breeding areas A1, A2. The spawning bed sheet collection container 62 is positioned on the downstream of the conveyor 20 closer to the other side of the conveyor 20 in the width direction (lower side in FIGS. 2 and 5). The spawning bed sheet collection container 62 is disposed to adjoin the leftover food collection container 61 in the width direction of the conveyor 20. When the spawning bed sheet 41 is conveyed by the belt 23, the spawning bed sheet collection container 62 receives the spawning bed sheet 41 dropped from the downstream end of the belt 23 and collects the spawning bed sheet 41 while folding it.

As shown in FIGS. 1 to 3 and 5, the protection sheet collection container 63 is disposed on the downstream side in the conveyance direction of the conveyor 20, and collects the protection sheet 42 discharged from the breeding areas A1, A2. The protection sheet collection container 63 is positioned on the downstream of the conveyor 20 closer to the other side of the conveyor 20 in the width direction (lower side in FIGS. 2 and 5). The protection sheet collection container 63 is disposed to adjoin the downstream side of the spawning bed sheet collection container 62. When the protection sheet 42 is conveyed by the belt 23, the protection sheet collection container 63 receives the protection sheet 42 dropped from the downstream end of the belt 23 and collects the protection sheet 42 while folding it.

The protection sheet collection container 63 collects the protection sheet 42 in a state in which the protection sheet 42 is separated from the spawning bed sheet 41. For example, the protection sheet collection container 63 includes a separation plate 63a disposed near the downstream end of the conveyor 20. The separation plate 63a guides only the protection sheet 42 separated from the spawning bed sheet 41 at the downstream end of the conveyor 20. The protection sheet collection container 63 collects only the protection sheet 42 separated and guided by the separation plate 63a.

### (4. Operation of Breeding Apparatus 1)

The operation of the breeding apparatus 1 will be described with reference to FIGS. 1 to 6. First, an operator disposes the spawning bed sheet holding member 51 on the frame 11 with the spawning bed sheet 41a wound around the spawning bed sheet holding member 51. The operator disposes the protection sheet holding member 52 on the frame 11 with the protection sheet 42a wound around the protection sheet holding member 52. The spawning bed sheet holding member 51 and the protection sheet holding member 52 are disposed on the upstream side of the conveyor 20.

Then, the operator pulls out a part of the leading end side of the spawning bed sheet 41a held by the spawning bed sheet holding member 51, and places the pulled out spawning bed sheet 41a on the upper surface of the belt 23 of the conveyor 20. The operator pulls out a part of the leading end side of the protection sheet 42a held by the protection sheet holding member 52, and lays the pulled out protection sheet 42a over the upper surface of the spawning bed sheet 41 on the upper surface of the belt 23 of the conveyor 20.

Then, the operator drives the conveyor 20 to convey the spawning bed sheet 41 and the protection sheet 42 laid over the upper surface of the belt 23 of the conveyor 20 to the downstream end in the conveyance direction of the conveyor 20. At this time, the spawning bed sheet 41 and the protection sheet 42 are conveyed along with the conveyance of the belt 23 by the frictional force between the belt 23 and the spawning bed sheet 41 and the frictional force between the spawning bed sheet 41 and the protection sheet 42.

Then, the operator puts the leading end of the spawning bed sheet 41 conveyed to the downstream end in the conveyance direction of the conveyor 20 into the spawning bed sheet collection container 62, and puts the leading end of the protection sheet 42 into the protection sheet collection container 63. That is, the operator separates the spawning bed sheet 41 and the protection sheet 42 and puts them into the spawning bed sheet collection container 62 and the protection sheet collection container 63, respectively.

In this way, the belt 23 of the conveyor 20, the spawning bed sheet 41, and the protection sheet 42 are laid one on top of another at a part of the conveyor 20 in the width direction (lower part in FIGS. 2 and 5) below the breeding areas A1, A2. The spawning bed sheet 41 is laid over the upper surface of the belt 23, and the protection sheet 42 is laid over the upper surface of the spawning bed sheet 41. The belt 23 of the conveyor 20 is exposed at the other part of the conveyor 20 in the width direction (upper part in FIGS. 2 and 5) below the breeding areas A1, A2.

Then, the operator carries adult breeding target organisms or larval breeding target organisms that will soon become adults into the breeding areas A1, A2. Instead of carrying the breeding target organisms by the operator, the breeding target organisms may be carried automatically or semi-automatically by a conveyance device (not shown) or by using the conveyor 20.

By controlling the control device in this state, the feeding device 30 supplies food to the breeding areas A1, A2. Specifically, the operator puts food in the hopper 31 of the feeding device 30, and the rotary brush 32 is driven by the control device, thereby supplying the food in the hopper 31 to the upper surface of the belt 23 of the conveyor 20. Simultaneously with or after the food supply to the upper surface of the belt 23, the control device drives the conveyor 20 to convey the food to the breeding areas A1, A2. After the food is conveyed to the breeding areas A1, A2, the drive of the conveyor 20 is temporarily stopped and the breeding target organisms can eat the food.

During the breeding period, the breeding target organism can move on the stay member, stay on the stay member, or get down on the upper surface of the belt 23. The breeding target organism moves to the food supply area on the upper surface of the belt 23 and eats food. When the breeding target organism spawns, as shown in FIG. 6, the organism moves to the spawning bed area where the spawning bed sheet 41 and the protection sheet 42 are laid and spawns eggs. At the time of spawning, the organism pierces the protection sheet 42 with its ovipositor to reach the position of the spawning bed sheet 41. Then, the organism spawns eggs on the spawning bed sheet 41. That is, the spawned eggs are held by the spawning bed sheet 41.

At the bottom surfaces of the breeding areas A1, A2, unwanted matter such as dust, excrement, and dead organisms in addition to the supplied food is present on the upper surface of the belt 23 that forms the food supply area. At the bottom surfaces of the breeding areas A1, A2, unwanted matter such as food, dust, excrement, and dead organisms is similarly present on the upper surface of the protection sheet 42 that forms the spawning bed area. The protection sheet 42 is laid over the upper surface of the spawning bed sheet 41. Therefore, the unwanted matter is present on the upper surface of the protection sheet 42 and adhesion of the unwanted matter to the spawning bed sheet 41 is suppressed.

The conveyor 20 is driven by the control device after a predetermined period has elapsed. Therefore, the parts that form the bottom surfaces of the breeding areas A1, A2 move downstream in the conveyance direction of the conveyor 20. Then, the unwanted matter present on the upper surface of the belt 23 constituting the food supply area is collected in the leftover food collection container 61.

Since the conveyor 20 is driven by the control device, the spawning bed sheet 41 with the spawned eggs is collected in the spawning bed sheet collection container 62. The operator can obtain the spawned eggs by periodically taking out the spawning bed sheet 41 collected in the spawning bed sheet collection container 62. Since the conveyor 20 is driven by the control device, the protection sheet 42 is collected in the protection sheet collection container 63 while being separated from the spawning bed sheet 41. The operator periodically takes out the protection sheet 42 collected in the protection sheet collection container 63. That is, the unwanted matter is collected together with the protection sheet 42 in the protection sheet collection container 63 because the spawning bed sheet 41 and the protection sheet 42 are collected separately. That is, the spawning bed sheet 41 is collected in the spawning bed sheet collection container 62 in a state in which the unwanted matter is separated.

As described above, the spawning bed sheet 41 is carried into the bottom positions of the breeding areas A1, A2 from the outside of the breeding areas A1, A2, and is carried to the outside from the bottom positions of the breeding areas A1, A2. In this way, the spawning bed can be disposed and collected by moving the spawning bed sheet 41. By repeating the movement of the spawning bed sheet 41, the spawning bed can be disposed and collected repeatedly. Thus, the spawning bed can be disposed and collected very easily. Since the protection sheet 42 can be moved together with the spawning bed sheet 41 while being laid over the spawning bed sheet 41, the spawning bed sheet 41 can always be kept clean.

### (5. Configuration of Breeding Apparatus 2 in Other Example)

The configuration of a breeding apparatus 2 in another example will be described with reference to FIG. 7. The breeding apparatus 1 described above has the food supply area and the spawning bed area on the bottom surfaces of the breeding areas A1, A2. In addition, the breeding apparatus 2 may have only the spawning bed area on the bottom surfaces of the breeding areas A1, A2. In this case, the spawning bed sheet 41 and the protection sheet 42 are disposed substantially over the entire upper surface of the belt 23 of the conveyor 20 in the width direction of the conveyor 20. In this case, it is appropriate to supply food by, for example, disposing a feeding container (not shown) on the stay member.

### Description of the Reference Numerals

1, 2 ... breeding apparatus, 10 ... breeding area forming member, 11 ... frame, 12, 13 ... breeding area facility, 20 ... conveyor, 21, 22 ... support roller, 23 ... belt, 30 ... feeding device, 31 ... hopper, 32 ... rotary brush, 41, 41a ... spawning bed sheet, 42, 42a ... protection sheet, 51 ... spawning bed sheet holding member, 52 ... protection sheet holding member, 61 ... leftover food collection container, 62 ... spawning bed sheet collection container, 63 ... protection sheet collection container, 63a ... separation plate, A1, A2 ... breeding area

## Claims

1. A breeding apparatus comprising:
a breeding area forming member that forms a breeding area for an organism and is open at least at a bottom of the breeding area; and
a spawning bed sheet that forms a bottom surface of the breeding area, has a belt shape, and is provided so as to be movable in a longitudinal direction of the belt shape relative to the breeding area forming member.

2. The breeding apparatus according to claim 1, further comprising a protection sheet that is laid over an upper surface of the spawning bed sheet, has a belt shape, and is provided so as to be movable in a longitudinal direction of the belt shape relative to the breeding area forming member together with the spawning bed sheet while being laid over the spawning bed sheet.

3. The breeding apparatus according to claim 2, wherein the protection sheet restricts passage of unwanted matter including at least a part of dust, food, excrement, and a dead body of the organism, and allows passage of an ovipositor of the organism.

4. The breeding apparatus according to any one of claims 1 to 3, further comprising a conveyor that is disposed below the breeding area and conveys the spawning bed sheet with the spawning bed sheet placed on the conveyor.

5. The breeding apparatus according to claim 4, wherein the spawning bed sheet is placed on a part of the conveyor in a width direction, and another part of the conveyor in the width direction serves as a food supply area.

6. The breeding apparatus according to claim 4 or 5, further comprising a spawning bed sheet holding member that is disposed on an upstream side in a conveyance direction of the conveyor and holds the spawning bed sheet before supply to the breeding area, wherein the spawning bed sheet is supplied to a position below the breeding area by being pulled out from the spawning bed sheet holding member by a conveyance force of the conveyor.

7. The breeding apparatus according to any one of claims 4 to 6, further comprising a spawning bed sheet collection container that is disposed on a downstream side in a conveyance direction of the conveyer and collects the spawning bed sheet discharged from the breeding area.

8. The breeding apparatus according to claim 2 or 3, further comprising:
a conveyor that is disposed below the breeding area and conveys the spawning bed sheet and the protection sheet with the spawning bed sheet and the protection sheet laid over the conveyor;
a spawning bed sheet holding member that is disposed on an upstream side in a conveyance direction of the conveyor and holds the spawning bed sheet before supply to the breeding area; and
a protection sheet holding member that is disposed on the upstream side in the conveyance direction of the conveyor and holds the protection sheet before supply to the breeding area, wherein
the spawning bed sheet is supplied to a position below the breeding area by being pulled out from the spawning bed sheet holding member by a conveyance force of the conveyor, and
the protection sheet is supplied to the position below the breeding area by being pulled out from the protection sheet holding member by the conveyance force of the conveyor.

9. The breeding apparatus according to claim 2 or 3, further comprising:
a conveyor that is disposed below the breeding area and conveys the spawning bed sheet and the protection sheet with the spawning bed sheet and the protection sheet laid over the conveyor;
a spawning bed sheet collection container that is disposed on a downstream side in a conveyance direction of the conveyer and collects the spawning bed sheet; and
a protection sheet collection container that is disposed on the downstream side in the conveyance direction of the conveyer and collects the protection sheet discharged from the breeding area while being separated from the spawning bed sheet.

10. The breeding apparatus according to any one of claims 4 to 9, wherein the conveyor is a belt conveyor.

## Amended claims

### Amended claims under Art. 19.1 PCT

1. (Currently amended) A breeding apparatus comprising:
a breeding area forming member that forms a breeding area for an organism and is open at least at a bottom of the breeding area;
a spawning bed sheet that forms a bottom surface of the breeding area, has a belt shape, and is provided so as to be movable in a longitudinal direction of the belt shape relative to the breeding area forming member; and
a protection sheet that is laid over an upper surface of the spawning bed sheet, has a belt shape, and is provided so as to be movable in a longitudinal direction of the belt shape relative to the breeding area forming member together with the spawning bed sheet while being laid over the spawning bed sheet.

2. (Currently amended) The breeding apparatus according to claim 1, wherein the protection sheet restricts passage of unwanted matter including at least a part of dust, food, excrement, and a dead body of the organism, and allows passage of an ovipositor of the organism.

3. (Currently amended) The breeding apparatus according to claim 1 or 2, further comprising a conveyor that is disposed below the breeding area and conveys the spawning bed sheet with the spawning bed sheet placed on the conveyor.

4. (Currently amended) The breeding apparatus according to claim 3, wherein the spawning bed sheet is placed on a part of the conveyor in a width direction, and another part of the conveyor in the width direction serves as a food supply area.

5. (Currently amended) The breeding apparatus according to claim 3 or 4, further comprising a spawning bed sheet holding member that is disposed on an upstream side in a conveyance direction of the conveyor and holds the spawning bed sheet before supply to the breeding area, wherein the spawning bed sheet is supplied to a position below the breeding area by being pulled out from the spawning bed sheet holding member by a conveyance force of the conveyor.

6. (Currently amended) The breeding apparatus according to any one of claims 3 to 5, further comprising a spawning bed sheet collection container that is disposed on a downstream side in a conveyance direction of the conveyer and collects the spawning bed sheet discharged from the breeding area.

7. (Currently amended) The breeding apparatus according to claim 1 or 2, further comprising:
a conveyor that is disposed below the breeding area and conveys the spawning bed sheet and the protection sheet with the spawning bed sheet and the protection sheet laid over the conveyor;
a spawning bed sheet holding member that is disposed on an upstream side in a conveyance direction of the conveyor and holds the spawning bed sheet before supply to the breeding area; and
a protection sheet holding member that is disposed on the upstream side in the conveyance direction of the conveyor and holds the protection sheet before supply to the breeding area, wherein
the spawning bed sheet is supplied to a position below the breeding area by being pulled out from the spawning bed sheet holding member by a conveyance force of the conveyor, and
the protection sheet is supplied to the position below the breeding area by being pulled out from the protection sheet holding member by the conveyance force of the conveyor.

8. (Currently amended) The breeding apparatus according to claim 1 or 2, further comprising:
a conveyor that is disposed below the breeding area and conveys the spawning bed sheet and the protection sheet with the spawning bed sheet and the protection sheet laid over the conveyor;
a spawning bed sheet collection container that is disposed on a downstream side in a conveyance direction of the conveyer and collects the spawning bed sheet; and
a protection sheet collection container that is disposed on the downstream side in the conveyance direction of the conveyer and collects the protection sheet discharged from the breeding area while being separated from the spawning bed sheet.

9. (Currently amended) The breeding apparatus according to any one of claims 3 to 8, wherein the conveyor is a belt conveyor.

10. (Canceled)
